# EUROPEAN PATENT SPECIFICATION

(11) **EP 1 879 509 B1**
(45) Date of publication and mention of the grant of the patent: **09.01.2013**
(21) Application number: 05739540.2
(22) Date of filing: 09.05.2005
(51) Int. Cl.: A61B 17/12

(54) **LIGATOR**
LIGATOR
LIGATEUR

(43) Date of publication of application: 23.01.2008
(73) Proprietor: EndoChoice, Inc., Alpharetta, GA 30009-2089 (US)
(72) Inventor: CHAMI, Salah, 3460 Birkerød (DK)
(74) Representative: Exell, Jonathan Mark
(86) International application number: PCT/DK2005/000312
(87) International publication number: WO 2006/119762

(56) References cited:
- WO-A2-02/45595
- US-A- 5 320 630
- US-A- 5 398 844
- US-A- 6 051 003
- US-A- 6 059 798

## Description

This invention relates to medical ligating instruments, and more particularly to devices used to ligate body tissue in a created or natural cavity in animals or in the human body, for example, varicose veins in the gullet or a duodenal diverticulum by means of individual elastic bands while observing the legations through an endoscope.

It takes supreme effort to manoeuvre an endoscope while you ligate internal tissue in a created or natural cavity in animals or in the human body for example varicose veins in the gullet or a duodenal diverticulum by means of individual elastic band while observing the legations through an endoscope if the surgeon is supposed to manoeuvre the endoscope and activate the release of a number of elastic band all by him self Therefore it is common practice an assistant helps the surgeon while he manoeuvre the endoscope and releases elastic bands during the actual ligation.

Furthermore it is very inconvenient for the patient when the endoscope is lead in to the gullet or other openings of the human body, therefore it most advantageous to perform a save and quick operation.

For known ligating instruments related to the present invention the trigger unit is not firmly fixed at the distal end the endoscope see for example US 6.149.659 which discloses the features of the preamble of claim 1. Therefore you must hold the trigger unit firmly with one hand, by the other hand you can draw the trigger cord to obtain the legation of the desired varix or diverticulum. A third and eventually a fourth hand are needed to hold and manoeuvre the endoscope.

For known ligating instruments related to the present invention 2 or more trigger cords are used in releasing the rubber bands see for example US 6.235.040 and WO 9716120. The consequence of having 2 or more trigger cord is that the cords can cross one and another and squeeze for example a varix which easily can result in a bleeding and put the patient health at stake and make the treatment very troublesome.

Therefore it is an object of the present invention to provide an improved ligating instrument where the surgeon very fast and with great safety can perform the operation by himself using a automatic trigger unit according to the invention which further if necessary can lead to saving of staff in connection with the surgery.

Further it is an object of the present invention to provide an improved ligating instrument where the great risk of squeezing body tissue for example varix in the gullet is eliminated in connection with the ligation.

The first mentioned object according to the present invention you can achieve using a ligating instrument, which include the means characterized in claim 1, whereby the trigger unit can be mounted firmly on the endoscope. Hereby is produced a one-man-operated endoscope, where the surgeon has complete control over the endoscope.

The grip of the trigger unit on the endoscope at the joint between the tube and the handle of the endoscope can be secured by means of a U-formed bracket on the connecting joint.

To enable the grip of the trigger unit on the endoscope at the joint between the tube and the handle of the endoscope no matter the diameter of the endoscope the connecting joint can be provided with a fixture for example a velcro (RTM) tape.

When the U-formed bracket is connected with the trigger unit by means of one or more toggle joint(s), you can secure the socket-shaped nozzle at the proximal end of the accessory channel of the endoscope and afterwards turn the U-formed bracket to fasten at the cylindrical joint between the tube and the handle of the endoscope.

It has surprisingly been revealed that it is possible to overcome the drawbacks which occur when trigger cords cross one another which can lead to squeezing of internal tissue such as a varix that consequently can lead to severe bleeding, when you have a single stranded trigger cord at the distale end of the trigger cord.

The use of a single stranded trigger cord implies an adequate big size of the knots or beads because a single knot or bead on the distale end of the trigger cord is responsible for that an elastic band is guided over the distale end of the bush and released for the ligation of a specific internal tissue.

When the trigger cord is manufactured of a metal wire, preferably a twisted metal wire, you achieve at least two essential advantages because a twisted metal wire simultaneous is rigid and very flexible, therefore the surgeon has an optimal touch with the firing of elastic bands and because of the big strength of metal wires very tiny dimension can be used which leave the optimally space in the accessory channel for the flushing if a bleeding occurs during the ligation.

The use of the one-man-operated, automatic, dual fixed, trigger unit according to the invention provides an adapted up winding of the trigger cord, corresponding to the release of a single elastic band at the distale end of the endoscope, said up winding, you can obtain by turning the revolving parts simultaneously to the built-in stop at their extreme position, where the release of an elastic band takes place and the simultaneous compressing of a built-in spring is performed. After the release of an elastic band has taken place the revolving part, at which the proximale end of the trigger cord is fixed, leaves its engagement with the part which, in an embodiment, has a winding track and automatically comes back to its starting point while the trigger cord is coiled up at said winding track.

When the trigger unit has a sound signal at the extreme position of the revolving parts, the surgeon instantly becomes aware of that the release of a elastic band has taken place, where after the surgeon instantly can let go of the revolving parts, where after the tensed revolving part automatically goes back to its starting point. Hereby is obtained the obvious advantage that the operation can be performed in a shorter amount of time which means less discomfort by the endoscope for the patient.

For flusching, if bleeding occurs during the ligation the trigger unit according to the invention has an integrated canal upon which a flexible injection tube can be mounted. When the injection tube is mounted the surgeon can flush the fiber optic from any angle at the proximale end of the endoscope by the means of a syringe.

By manufacturing of a bush according to the invention, there is provided a retainer for the elastic bands by the use of a single stranded trigger cord. The use of a single stranded trigger cord leads to a safes operation for the patient and more over said bush charged with elastic band is considerably easier and therefore less expensive to produce.

A bush charged with elastic band may to prepared to mesh with the distale end of an endoscope after joining the trigger cord from the bush with the trigger cord from the trigger unit via the accessory channel of the endoscope.

Description of the drawing according to the disclosure where:
fig.1 is a perspective view of a trigger unit,
fig.2 is a perspective view of a trigger unit where a trigger cord, is guided into the trigger unit,
fig. 3 is a longitudinal section through a bush which upon is placed flexible trigger cord with beads and elastic band and
fig. 4 is a perspective view of a trigger unit according to the present disclosure firmly mounted on the proximale end of an endoscope.

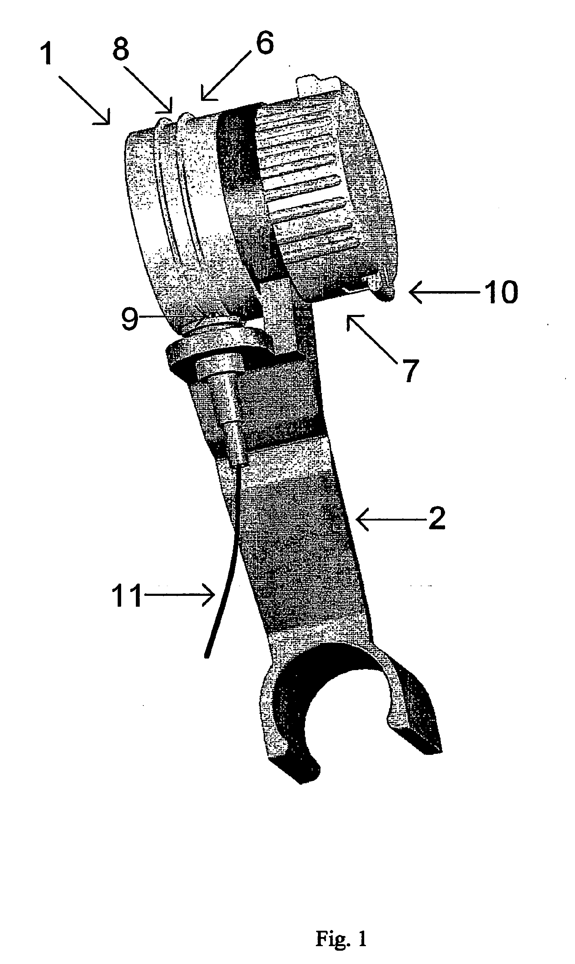

Fig. 1 illustrates a trigger unit, which in general is named with the reference number 1. It has a connecting part 2, on which a socket-shaped nozzle 3 is mounted, further a U-formed bracket 4 is mounted on the connecting part 2 by means of a toggle joint 5. One of the two parts 6 and 7 can revolve together on a shaft, not shown, since the shaft is lead through a bedding, not shown, in the connecting part 2. The first part 6 has a winding track 8 which is meant for the guiding and winding of the trigger cord 11. The other revolving part 7 has a built-in spring, which after the release of an elastic band 19 ensure, that the revolving part 7 leaves its engagement with part 6 and automatically comes back to its starting point, while the part 6 ensures automatic winding of the trigger cord 11 on the winding track 8. From the socket-shaped nozzle 3 the trigger cord 11 is lead through the hole 9 in the winding track 8 and through the trigger units part 6, part 2 and part 7 and further to the cord clamp 10 where the cord is secured. The cord clamp 10 is shown in its locked position on fig. 1.

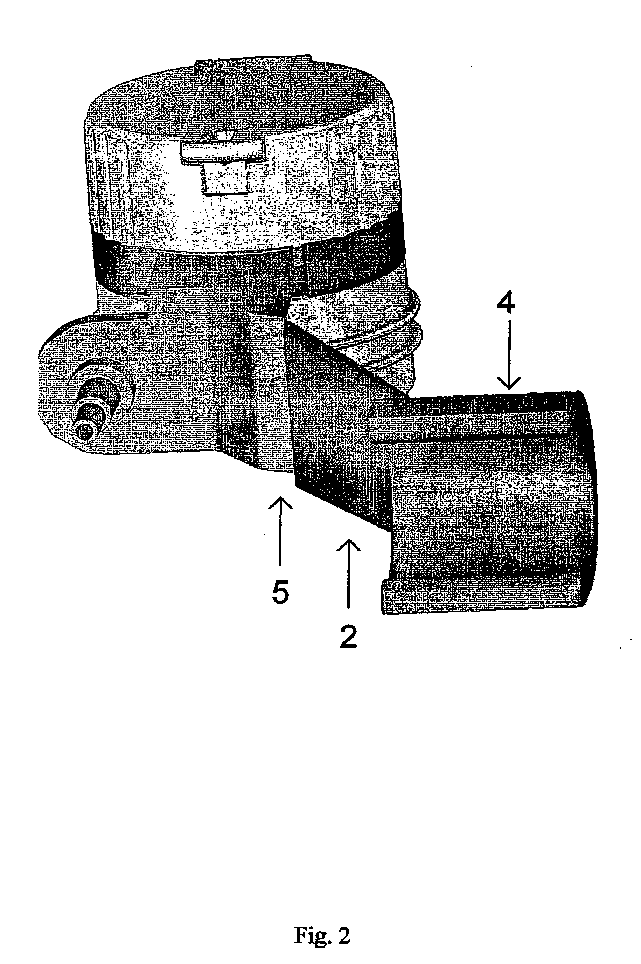

Fig. 2 illustrates a trigger unit 1 seen from below, where the U-formed bracket 4 is connected with the trigger unit 1 by means of one or more toggle joint(s) 5.

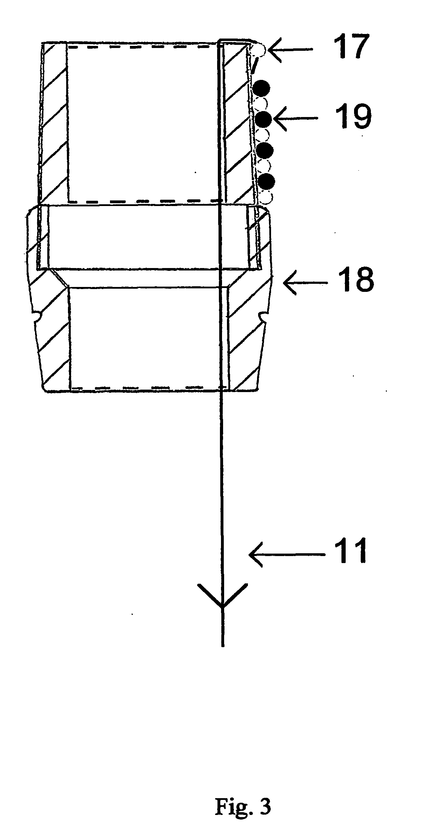

Fig. 3 illustrates how the distale end of the trigger cord 11 is supplied with a number of beads 17. The trigger cord 11 is, as shown, guided through a bush 18 and folded backwards on the outer end of the bush. The bush is mounted on the distale end of the tube 14 of the endoscope 1. Elastic band 19 is placed so they encircle the bush 18 and the trigger cord 11 in between the beads 17.

Therefore it is obvious, if the trigger cord 11 is pulled inwards into the tube of the endoscope 4, meaning downwards on fig. 3, the beads 17 on the distale end of the trigger cord 11 will guide the elastic bands 19 over the distale end of the bush 18 and successively release the elastic bands 19.

The pull of the trigger cord 11 into the tube of the endoscope 4 is achieved when the proximale end of the trigger cord 11 is fastened at the winding track 8 on the trigger unit 1 by means of the cord clamp 10. Hereby can the revolving parts revolve from the starting position to their extreme position where there is a built-in stop, during which the release of a elastic bands 19 takes place.

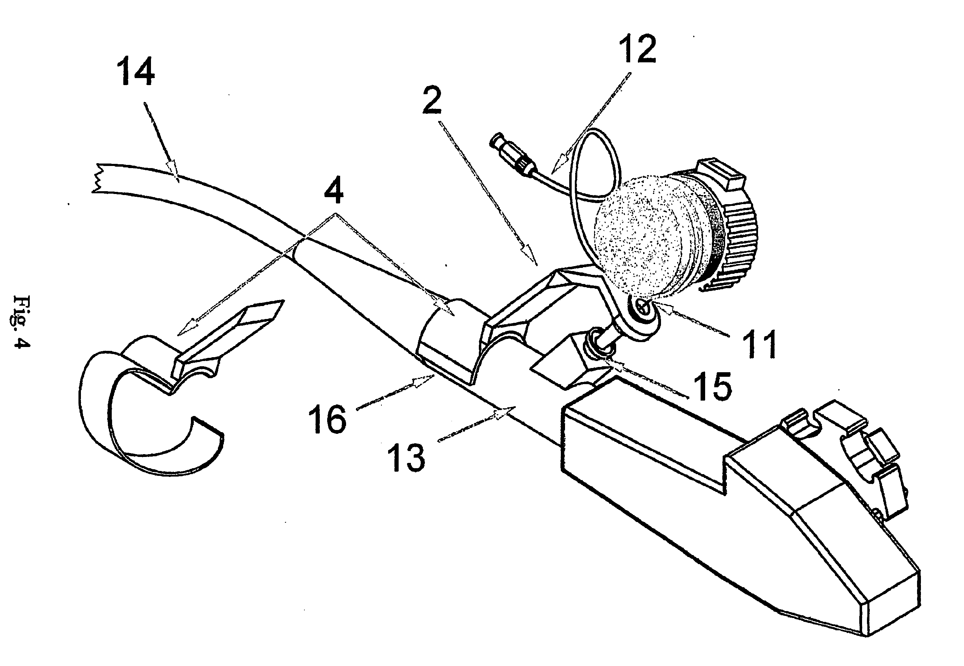

Fig. 4 illustrates a trigger unit mounted on the proximal end of an endoscope. The bigger cord 11 is guided from the proximal opening 15 of the endoscope to the trigger unit 1, on which a flexible injection tube 12 can be fixed. The dual fixed trigger unit 1 is partly fixed by the anchoring of the socket-shaped nozzle 3 on the trigger unit 1 into the accessory channel 15 of the endoscope and partly by the connecting unit 2 on the trigger unit 1 via a U-formed bracket 4.

When operating, the distale end of the endoscope which is supplied with a loaded bush 18 is guided over a varicose vein, while said vein is observe through the endoscope or on a monitor. If it is necessary the varicose vein can be sucked into the bush 18 using a pump.

When the varicose vein is in place, which can be observed through the endoscope or on a monitor an elastic band 19 can be released, said band then places itself around the varicose vein, which leads to halt of the blood circulation and consequently to necrosis of the tissue. When the distal end of the endoscope is provided with a loaded bush, one or more varicose vein(s) can be treated in the same manner, as long as there is an elastic band on the bush 18. In this way individual elastic band 19 can be placed around separate varicose veins.

## Claims

1. A ligator for use with an endoscope having a handle, a tube and an accessory channel, the ligator comprising:
a trigger unit (1) including first and second revolving parts (6, 7) and a connecting part (2) extending from the trigger unit and adapted to mount the trigger unit to the handle of the endoscope;
a socket-shaped nozzle (3) adapted to be received within the accessory channel (15) of the endoscope;
a bush (18) adapted to mount to an end of the tube of the endoscope, the bush (18) supporting elastic bands (19) to be deployed for ligation; and
a trigger cord (11) connected at a first end to the second revolving part (7) of the trigger unit and connected at a second end to the bush (18);
wherein the first revolving part (6) is arranged to cause up-winding of the trigger cord(11), **characterised in that** the first and second revolving parts (6, 7) are adapted to cause deployment of an elastic band (19) when they are turned together from a starting position to an extreme position where there is a built-in stop, wherein once the elastic band (19) has been deployed the second revolving part (7) is configured to leave its engagement with the first revolving part (6), which remains stationary, and automatically returns to the starting position.

2. The ligator of claim 1, wherein the revolving parts (6, 7) are further adapted to sound a signal at the extreme position to signal a user that release of the elastic band (19) has occurred.

3. The ligator of claim 1, wherein the revolving parts (6, 7) include a cord clamp (10) that secures the first end of the trigger cord (11) to the second revolving part (7).

4. The ligator of claim 1, wherein the first revolving part (6) includes a winding track (8) that guides the trigger cord.

5. The ligator of claim 4, wherein the first revolving part (6) is adapted to turn when the second revolving part (7) is turned and to coil the trigger cord (11) on the winding track (8).

6. The ligator of claim 1, wherein the connecting part (2) is elongated.

7. The ligator of claim 1, wherein the connecting part (2) comprises a U-shaped bracket adapted to grip the handle of the endoscope.

8. The ligator of claim 1, wherein the connecting part (2) comprises at least one toggle joint.

9. The ligator of claim 1, wherein the bush (18) comprises an outer surface and defines an inner passage.

10. The ligator of claims 4 and 9 wherein the elastic bands (19) are wrapped around the outer surface of the bush (18).

11. The ligator of claims 5 and 9, wherein the trigger cord (11) extends from the outer surface and into the inner passage of the bush (18).

12. The ligator of claim 11, wherein the trigger cord (11) comprises beads (17) provided along at least a part of its length.

13. The ligator of claim 11, wherein the trigger cord (11) extends from the bush (18), through the nozzle (3), and into the revolving parts (6, 7).

14. The ligator of claim 1, further comprising a flexible injection tube (12).

## Patentansprüche

1. Ligator zur Verwendung bei einem Endoskop mit einem Handgriff, einem Rohr und einem Zugangskanal, wobei der Ligator aufweist:
eine Auslöseeinheit (1) einschließend erste und zweite drehende Teile (6, 7) und ein Verbindungsteil (2), welches von der Auslöseeinheit ausgeht und so aufgebaut ist, dass es die Auslöseeinheit an dem Handgriff des Endoskopes befestigt;
eine büchsenförmige Kabeltülle (3), die so aufgebaut ist, dass sie in dem Zugangskanal (15) des Endoskops aufnehmbar ist;
eine Hülse (18), die so aufgebaut ist, dass sie an einem Ende des Rohrs des Endoskops befestigbar ist, wobei die Hülse (18) elastische Bänder (19) trägt, die für eine Ligation eingesetzt werden; und
ein Auslöseband (11), das mit einem ersten Ende an dem zweiten drehenden Teil (7) der Auslöseeinheit angeschlossen ist und mit einem zweiten Ende an der Hülse (18) angeschlossen ist;
wobei das erste drehende Teil (6) so angeordnet ist, dass es ein Aufwickeln des Auslösebandes (11) verursacht,
**dadurch gekennzeichnet,**
**dass** die ersten und zweiten drehenden Teile (6, 7) so aufgebaut sind, dass sie einen Einsatz des elastischen Bandes (19) veranlassen, wenn sie gemeinsam aus einer Startposition in eine Extremposition gedreht werden, in welcher ein eingebauter Halt vorgesehen ist,
wobei dann, wenn das elastische Band (19) eingesetzt ist, das zweite drehende Teil (7) so aufgebaut ist, dass es den Eingriff mit dem ersten drehenden Teil (6) verlässt, welcher stationär bleibt, und automatisch in die Startposition zurückdreht.

2. Ligator nach Anspruch 1,
in welchem die drehenden Teile (6, 7) außerdem so angepasst sind, dass sie ein Signal an der Extremposition hörbar machen, um einem Benutzer zu signalisieren, dass eine Freigabe des elastischen Bandes (19) stattgefunden hat.

3. Ligator nach Anspruch 1,
in welchem die drehenden Teile (6, 7) eine Bandklemme (10) einschließen, welche das erste Ende des Auslösebandes (11) an dem zweiten drehenden Teil (7) befestigt.

4. Ligator nach Anspruch 1,
in welchem der erste drehende Teil (6) eine Aufwickelstrecke (8) einschließt, die das Auslöseband führt.

5. Ligator nach Anspruch 4,
in welchem das erste drehende Teil (6) so aufgebaut ist, dass es dreht, wenn das zweite drehende Teil (7) gedreht wird, und das Auslöseband (11) auf der Aufwickelstrecke (8) aufspult.

6. Ligator nach Anspruch 1,
in welchem das Verbindungsteil (2) länglich ist.

7. Ligator nach Anspruch 1,
in welchem das Verbindungsteil (2) eine U-förmige Klammer aufweist, die aufgebaut ist, um den Handgriff des Endoskops zu greifen.

8. Ligator nach Anspruch 1,
in welchem das Verbindungsteil (2) zumindest eine Hebelverbindung aufweist.

9. Ligator nach Anspruch 1,
in welchem die Hülse (18) eine Außenoberfläche aufweist und einen inneren Durchgang definiert.

10. Ligator nach Anspruch 4 und 9,
in welchem die elastischen Bänder (19) um die äußere Oberfläche der Hülse (18) herumgewickelt sind.

11. Ligator nach Anspruch 5 und 9,
in welchem das Auslöseband (11) von der äußeren Oberfläche ausgeht und sich hinein in den inneren Durchgang der Hülse (18) erstreckt.

12. Ligator nach Anspruch 11,
in welchem das Auslöseband (11) Riffelungen (17) aufweist, die längs zumindest eines Teils der Länge vorgesehen sind.

13. Ligator nach Anspruch 11,
in welchem das Auslöseband (11) sich von der Hülse (18) durch die Kabeltülle (3) hinein in die drehenden Teile (6, 7) erstreckt.

14. Ligator nach Anspruch 1,
außerdem aufweisend eine flexible Injektionsröhre (12).

## Revendications

1. Ligateur à utiliser avec un endoscope comprenant une poignée, un tube et un canal à accessoire, le ligateur comprenant:
une unité de déclenchement (1) comprenant des première et deuxième parties rotatives (6, 7) et une partie de connexion (2) qui s'étend à partir de l'unité de déclenchement et qui est adaptée pour monter l'unité de déclenchement sur la poignée de l'endoscope;
une buse en forme de manchon (3) adaptée pour être reçue à l'intérieur du canal à accessoire (15) de l'endoscope;
une douille (18) adaptée pour être montée sur une extrémité du tube de l'endoscope, la douille (18) supportant des bandes élastiques (19) à déployer pour réaliser une ligature; et
un câble de déclenchement (11) qui est connecté par une première extrémité à la deuxième partie rotative (7) de l'unité de déclenchement et qui est connecté par une deuxième extrémité à la douille (18),
dans lequel la première partie rotative (6) est agencée de manière à entraîner l'enroulement ascendant du câble de déclenchement (11),
**caractérisé en ce que** les première et deuxième parties rotatives (6, 7) sont adaptées pour entraîner le déploiement d'une bande élastique (19) lorsqu'elles tournent ensemble à partir d'une position de départ jusqu'à une position extrême où un arrêt intégré est prévu, dans lequel, une fois que la bande élastique (19) a été déployée, la deuxième partie rotative (7) est configurée de manière à sortir de son engagement avec la première partie rotative (6), qui reste stationnaire, et à retourner automatiquement dans la position de départ.

2. Ligateur selon la revendication 1, dans lequel les parties rotatives (6, 7) sont en outre adaptées pour provoquer un signal sonore à la position extrême afin de signaler à un utilisateur qu'il s'est produit un relâchement de la bande élastique (19).

3. Ligateur selon la revendication 1, dans lequel les parties rotatives (6, 7) comprennent un serre-câble (10) qui fixe la première extrémité du câble de déclenchement (11) à la deuxième partie rotative (7).

4. Ligateur selon la revendication 1, dans lequel la première partie rotative (6) comprend un rail d'enroulement (8) qui guide le câble de déclenchement.

5. Ligateur selon la revendication 4, dans lequel la première partie rotative (6) est adaptée pour tourner lorsque la deuxième partie rotative (7) tourne et pour bobiner le câble de déclenchement (11) sur le rail d'enroulement (8).

6. Ligateur selon la revendication 1, dans lequel la partie de connexion (2) est allongée.

7. Ligateur selon la revendication 1, dans lequel la partie de connexion (2) comprend une console en forme de U qui est adaptée pour saisir la poignée de l'endoscope.

8. Ligateur selon la revendication 1, dans lequel la partie de connexion (2) comprend au moins une articulation à genouillère.

9. Ligateur selon la revendication 1, dans lequel la douille (18) présente une surface extérieure et définit un passage intérieur.

10. Ligateur selon les revendications 4 et 9, dans lequel les bandes élastiques (19) sont enroulées autour de la surface extérieure de la douille (18).

11. Ligateur selon les revendications 5 et 9, dans lequel le câble de déclenchement (11) s'étend à partir de la surface extérieure et dans le passage intérieur de la douille (18).

12. Ligateur selon la revendication 11, dans lequel le câble de déclenchement (11) comprend des perles (17) qui sont prévues le long d'au moins une partie de sa longueur.

13. Ligateur selon la revendication 11, dans lequel le câble de déclenchement (11) s'étend à partir de la douille (18), à travers la buse (3) et dans les parties rotatives (6, 7).

14. Ligateur selon la revendication 1, comprenant en outre une tube d'injection flexible (12).
